(19)

Europäisches Patentamt
European Patent Office
Office européen des brevets

(11)  **EP 2 792 409 B1**

(12)  **EUROPEAN PATENT SPECIFICATION**

(45) Date of publication and mention
of the grant of the patent:
**22.01.2020  Bulletin 2020/04**

(21) Application number: **12858403.4**

(22) Date of filing: **14.11.2012**

(51) Int Cl.:
*B01J 37/00* (2006.01)     *B01J 23/28* (2006.01)
*B01J 37/04* (2006.01)     *B01J 35/02* (2006.01)
*B01J 27/20* (2006.01)     *B01J 27/18* (2006.01)
*B01J 27/051* (2006.01)    *C07C 1/04* (2006.01)
*C07C 9/04* (2006.01)      *C01G 39/06* (2006.01)

(86) International application number:
**PCT/KR2012/009569**

(87) International publication number:
**WO 2013/089353 (20.06.2013 Gazette 2013/25)**

(54) **METHOD FOR PREPARING A DIRECT METHANATION CATALYST FOR SYNTHETIC GAS**

VERFAHREN ZUR HERSTELLUNG EINES DIREKTMETHANISIERUNGSKATALYSATORS FÜR SYNTHETISCHES GAS

PROCÉDÉ DE PRÉPARATION D'UN CATALYSEUR DE MÉTHANATION DIRECTE POUR GAZ SYNTHÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2011   KR 20110136530**

(43) Date of publication of application:
**22.10.2014   Bulletin 2014/43**

(73) Proprietor: **Korea Institute of Energy Research**
**Daejeon 305-343 (KR)**

(72) Inventors:
• **KIM, Seong-Soo**
**Daejeon 305-721 (KR)**
• **SHIN, Dae-Hyun**
**Daejeon 305-370 (KR)**
• **KIM, Jin-Gul**
**Asan-si**
**Chungcheongnam-do 336-040 (KR)**

(74) Representative: **Staeger & Sperling**
**Partnerschaftsgesellschaft mbB**
**Sonnenstraße 19**
**80331 München (DE)**

(56) References cited:
EP-A1- 0 133 031          WO-A1-2012/040111
KR-A- 20100 053 617       KR-B1- 100 586 163
US-A- 4 243 554           US-A- 4 243 554
US-A- 6 156 693           US-A1- 2005 059 545
US-A1- 2010 304 964       US-B1- 6 451 729

EP 2 792 409 B1

**Description**

**Technical Field**

The present invention relates to a method for preparing a direct methanation catalyst for a synthetic gas.

**Background Art**

Main component of natural gas is $CH_4$, and it is a clean fuel producing almost no $SO_2$ and much less $NO_x$, dust, $CO_2$, and the like than coal or oil, therefore the demand thereof is continuously increasing globally. However, since it is anticipated that the supply of natural gas may not suffice the demand in future, catalytic methanation technologies, producing synthetic gas containing $H_2$ and CO through gasification of carbon containing materials such as coal, biomass and the like, and producing synthetic natural gas (SNG) through methanation of the produced synthetic gas using catalysis, are receiving renewed attention from worldwide.

Technologies for catalytic methanation of synthetic gas had already begun to be studied actively in the United States due to the gas crisis in the 1970s and expensive natural gas price; the first commercial plant in the world, producing synthetic natural gas from synthetic gas produced through gasification of coal, was built in Great Plains of North Dakota State of the United States in 1984, and has been operating up to now since then.

In a conventional catalytic methanation process, the most commonly used catalyst is Ni series catalyst. Methanation of synthetic gas when using Ni series catalyst occurs through the following reaction.

<Reaction formula 1> $\qquad$ $CO + 3H_2 \rightarrow CH_4 + H_2O$ $\Delta H°_{298}$ = -206 kJ/mol

In the above Reaction formula 1, the $H_2/CO$ ratio is 3. However, $H_2/CO$ ratio of a synthetic gas produced during gasification of coal or biomass is generally less than or equal to 2. If $H_2/CO$ ratio is less than or equal to 3, the selectivity of a catalyst is getting lower and carbon deposition over the catalyst is occurring. Thus, in order to maintain $H_2/CO$ ratio at 3 in the Reaction formula 1, $H_2/CO$ produced in the water gas Reaction formula 2 as described below, must be supplied as a raw material.

<Reaction formula 2> $\qquad$ $CO + H_2O \rightarrow CO_2 + H_2$ $\Delta H°_{298}$ = -41 kJ/mol

As described above, a conventional catalytic methanation process is a two stage reaction process comprised of a methanation reaction represented as Reaction formula 1 and a water gas reaction represented as the Reaction formula 2.

Other than the conventional catalyst methanation process, catalyst methanation may occur by the Reaction formula 3 as described below so called direct methanation process.

<Reaction formula 3> $\qquad$ $2H_2 + 2CO \rightarrow CH_4 + CO_2$ $\Delta H°_{298}$ = -247 kJ/mol

The conventional catalyst methanation process using Ni series catalyst requires a large amount of $H_2O$, as shown in the Reaction formula 2, in order to produce $H_2$ to be used in the Reaction formula 1, and the required $H_2O$ is being used by re-circulating the $H_2O$ produced in the Reaction formula 1. Therefore, addition of the high cost $H_2O$ re-circulation process to the conventional catalyst methanation process becomes a factor which reduces economics of the process. However, since the direct methanation reaction does not require a $H_2O$ re-circulation process, as shown in the Reaction formula 3, process can be simplified and the economics thereof can be improved.

The reactivity of a catalyst for methanation of a synthetic gas may be evaluated by CO conversion rate as defined Equation 1 as described below.

<Equation 1>

$$CO \text{ conversion rate} = \Sigma n_i M_i / M_{CO}$$

In the above Equation 1, $M_i$ means the concentration (volume %) of chemical species i, $n_i$ means the number of carbon atoms contained in the chemical species among the produced materials, and $M_{CO}$ means the concentration (volume %) of CO among the produced materials.

A conventional catalyst for methanation containing Ni series catalyst requires installation of an apparatus for removing acid gases at the front of the catalyst reactor since the performance of the catalyst is degraded due to the poisoning thereof easily by the toxic gases such as ammonia, hydrogen sulfide, and the like contained in the synthetic gas. In a direct methanation reaction, sulfides of transition metals such as V, W, Mo, and the like are frequently used as a catalyst; and since these sulfides of transition metals have sulfur resistance, it is advantageous in that installation of an acid gas removing process prior to the methanation process is not necessary. Other than methanation reaction, transition metal catalysts have been used for various usages such as Fischer-Tropsch reaction, denitrification reaction, desulfurization reaction, reforming reaction, decomposition reaction, coal liquefaction, water gas reaction, and the like.

Preparation of a transition metal catalyst in many cases relies on impregnation and precipitation on the support material. Although precipitation is commonly used, it has the disadvantage that catalysts can only be usable after undergoing an activation process. Moreover, there is a disadvantage that the dispersivity and the distribution of the activated particles not necessarily increased even a support having large surface is used. It is known that the successive activation process causes changes in the property and the cohesion of the activated particles distributed on the support material.

US-A-4,243,554 and WO-A-2012/040111 disclose the preparation of MoS2.

$MoS_x$ which is frequently used in hydrogenation process, WGS process, and methanation process is known to have S/Mo ratio of 1.5 to 2.5. According to the result of a recent study, it is reported that a $MoS_2$ crystal having smaller stacked atomic layers has superior activation reaction in hydrogenation decomposition process than a $MoS_2$ crystal having higher stacked atomic layers. According to the result of a XRD analysis, it is shown that the higher the treatment temperature, the size of the crystal increases, thus the surface area decreases and the number of stacked layers of $MoS_2$ increases, therefore the length of the stacked layers are increased. The reasons are: firstly, a $MoS_x$ is unstable at the temperature above 400°C even in a saturated $H_2S$ gas environment; and secondly, an increase in the treatment temperature causes thermal degradation of the $MoS_x$ regardless of the gas types.

It is shown that $MoS_{2-x}C_x$ with an increased carbon concentration is produced after the HDS reaction in the organic solvent at the reaction pressure of 35 atm, the dispersivity and the specific surface area of the catalyst prepared according to such process is increased compared with the catalyst prepared at conventional 1 atm, and has 7 times superior activation reaction compared with the catalyst prepared at a normal pressure.

It is assumed that if a solvent is used when synthesizing a $MoS_x$, carbon is deposited on the $MoS_2$ particles and isolates them and plays a role in increasing the dispersivity thereof, and forms a carbide surface coating on the surface of the $MoS_x$, which is an activation point; and the organic sulfide acts like a thermal well and plays a role in decreasing the effect of the exothermic reaction occurring during the sulfurization of the oxide precursors, thereby preventing thermal degradation of the catalysts due to the increase in the synthetic heat.

It was reported that the sulfurized metal catalyst containing carbon stably performs DBT desulfurization reaction more than 1,000 hours under the condition of 350°C and 35atm. In addition, it is also reported that the edge area of the $MoS_2$, the carbon concentration, and the reaction activation are increasing in a proportionate relation with each other.

From the investigation using IR, it is reported that since carbon is contained in $MoS_2$ during decomposition of ammonium tetrathiomolybdate (ATM), the peak intensity at 385cm$^{-1}$ is decreasing; through the investigation using TEM and XRD, it is observed that the size of the newly prepared $MoS_2$ specimen is decreased from 94nm to 47nm carbon containing $MoS_2$ due to the inclusion of carbon.

When comparatively reviewing on the various foregoing methods for preparing catalysts, it is understood that transition metal sulfides manufactured under the high pressure, especially $MoS_x$ shows increase in dispersivity and specific surface area, high reaction activation, poison resistance, and reaction durability; thus, it is needed to develop a transition metal sulfide catalyst manufactured under the high pressure. Furthermore, it is expected that medium sized air voids (meso pore distribution) can be developed by adjusting the size of the air voids inside the catalyst powder through the physical high pressure compression process.

## Summary of Invention

### Technical Problem

An objective of the present invention is to provide a method for preparing a catalyst for direct synthetic gas methanation, which can improve direct synthetic gas methanation efficiency, by preparing $MoS_2$ series catalysts adopting a high pressure synthesizing technique.

### Solution to Problem

To achieve the above described objective, the present invention provides a method for preparing a direct methanation catalyst for a synthetic gas, as a method for preparing a molybdenum disulfide (MoS2) series direct methanation catalyst, being characterized in that and including the steps of: pulverizing step for pulverizing precursors containing sulfur and molybdenum to a size of less than 10 mesh (2.00 mm); mixing step for mixing a material containing solvent into the precursors pulverized in said pulverizing step; reacting step for preparing a catalyst by disposing said material mixed in said mixing step inside a reactor, and letting it react under the high pressure $H_2$ gas environment of 10 bar to 200 bar; and activating step for activating the prepared catalyst for activation thereof, wherein the solvent is added 0.01 to 100 parts by weight for 1 part by weight of the precursor.

According to an exemplary embodiment, said reacting step may be the one, wherein $H_2$ gas is allowed to flow continuously passing through the inside of the reactor using a mass flow meter, and the internal pressure of the reactor is maintained 10bar to 200bar using a back pressure regulator, then the temperature is raised to 300-400°C, and subjected to reaction for 2-4 hours.

The flow rate of said $H_2$ gas is preferably 1cc/min to 300cc/min per 5g of precursors.

According to another exemplary embodiment, said reacting step may be the one, wherein $H_2$ gas is allowed to flow continuously passing through the inside of the reactor using a check valve, and the internal pressure of the reactor is maintained 10bar to 200bar using a back pressure regulator, then the temperature is raised to 300-400°C, and subjected to reaction for 2-4 hours.

According to yet another exemplary embodiment, said reacting step may be the one, wherein $H_2$ gas is being injected into the inside of the reactor, and the pressure is maintained 10bar to 200bar, then the temperature is raised to 300-400°C, and subjected to reaction for 2-4 hours.

Said precursor may be ammonium tetrathiomolybdate (ATM).

Said solvent may include at least one selected from the group comprising water, cyclohexane, acetone, heptane, hexadecane, methylnaphthalene, decaline, and tetralin. Said solvent is added 0.01 to 100 parts by weight for 1 part by weight of the precursor.

The material in said mixing step may further include a co-catalyst. Said co-catalyst may be a metal or a metal compound selected from the group comprising Zr, Sm, Ce, Yr, Yb, Si, and Al. Said co-catalyst is preferably added 0.01 to 20 parts by weight for 1 part by weight of the precursor.

Said pulverizing step may be the one wherein precursors are coarsely grinded, then a pressure 5bar to 40,000bar is being applied thereon, and then they are re-pulverized into the size of less than 10 mesh (2.00 mm).

The material in said mixing step may further contain sulfur component therein. Said sulfur component may include at list one selected from the group comprising DMDS, S, DBT, butanethiol, and $NH_4CNS$. Said sulfur component may be added 0.01 to 100 parts by weight for 1 part by weight of the precursor.

In order to achieve other objective of the present invention, the present invention provides a method for preparing a direct methanation catalyst for a synthetic gas being characterized in that it is prepared by the above described preparation method.

## Advantageous Effects of Invention

The above described direct methanation catalyst for a synthetic gas prepared according to the present invention may achieve effect of cost down more than 30% in process installation and operation thereof since there is no need for a separate installation of an apparatus for removing acid gases and a water gas reactor at the front end of the methanation process.

Especially the catalyst prepared according to the present invention shows increase in dispersivity and specific surface area, high reaction activation, poison resistance, and reaction durability, thus there is an effect of improving the direct synthetic gas methanation efficiency.

## Description of Embodiments

Hereinafter the present invention will further be described more in detail as follows.

In a method for preparing a molybdenum disulfide ($MoS_2$) series catalyst for direct synthetic gas methanation, the present invention provides a method for preparing a direct methanation catalyst for a synthetic gas, including the steps of: pulverizing step for pulverizing precursors containing sulfur and molybdenum to a size of less than 10 mesh (2.00 mm); mixing step for mixing a material containing solvent into the precursors pulverized in said pulverizing step; reacting step for preparing a catalyst by disposing said material mixed in said mixing step inside the reactor, and letting it react under the high pressure $H_2$ gas environment of 10 bar to 200 bar; and activating step for activating the prepared catalyst for activation thereof, wherein the solvent is added 0.01 to 100 parts by weight for 1 part by weight of the precursor.

In addition, the present disclosure provides a direct methanation catalyst for a synthetic gas prepared according to the above described preparation method.

A direct methanation catalyst for a synthetic gas prepared according to the present invention will further be described more in detail through a method for preparing thereof as follows.

In order to prepare a direct methanation catalyst for a synthetic gas according to the present invention, it undergoes a pulverizing step first. Said pulverizing step is a step for pulverizing precursors containing sulfur and molybdenum to a size less than 10 mesh (2.00 mm).

Said precursor is based on $MoS_2$, i.e. containing sulfur and molybdenum; it may be used without limitation if it is used for preparing a direct methanation catalyst for a synthetic gas. More preferably, said precursors may be an ammonium tetrathiomolybdate (ATM).

Said pulverizing is performed in order to increase the reactivity in the after-mentioned reacting step; according to the invention the pulverizing grain-size in said pulverizing step is less than 10 mesh (2.00 mm) considering the efficiency of the process.

Preferably, said pulverizing step may be the one wherein the precursors are coarsely grinded, then a pressure 5bar to 40,000bar is being applied thereon, and then they are re-pulverized into the size of less than 10 mesh (2.00 mm).

In the above described re-pulverization, the pressure being applied to the coarsely grinded precursor powder is 5bar to 40,000bar; if the precursor powder is re-pulverized after applying pressure to the coarsely grinded precursor powder in the above described range, the size distribution becomes uniform since the irregular air voids are destroyed during the process wherein the particle size of the precursor powder becomes finer; thus the improvement in the yield of the methanation of prepared catalyst may be maximized. At this time, application of the pressure to said coarsely grinded precursor powder for at least 5 min is sufficient. It is preferred that the grain-size for pulverizing in said re-pulverizing to be less than 10 mesh (2.00 mm), more preferably, the size of 1 mesh (25.4 mm) to 10 mesh (2.00 mm).

Said mixing step is a step wherein material containing solvent is being mixed into the precursors pulverized in said pulverizing step.

Said solvent is being added for effective occurrence of the gaseous reaction by dissolving the precursors. Water, organic solvent or the mixture thereof may be used for said solvent. Said organic solvent increases the boiling point, and when it is included in the catalyst, the carbon contained therein prevents thermal degradation. Said organic solvent may include at least one selected from the group comprising water, cyclohexane, acetone, heptane, hexadecane, methylnaphthalene, decaline, and tetralin. Said solvent is added 0.01 to 100 parts by weight for 1 part by weight of the precursor.

Said solvent is added 0.01 to 100 parts by weight for 1 part by weight of the precursor. When said solvent is added less than 0.01 parts by weight for 1 part by weight of the precursor, there is a problem wherein the efficiency of the prepared catalyst is decreasing since the reaction is not sufficiently occurred due to the insufficient dissolving of the precursor; if it is exceeded 100 parts by weight, there is a problem wherein the reactivity is decreasing since the contact efficiency between the precursors and the gases is reduced.

In said mixing step a co-catalyst may be added to improve the reactivity and the stability.

For said co-catalyst, a generally used one in the field of the art may be applied, and preferably at least a metal or a metal compound selected from the group comprising Zr, Sm, Ce, Yr, Yb, Si, and Al, may be included.

Said co-catalyst may be added 0.01 to 100 parts by weight for 1 part by weight of the precursor. When said co-catalyst is added less than 0.01 parts by weight for 1 part by weight of the precursor, there is a problem wherein the additive effect of the co-catalyst is not sufficiently occurring; if it is exceeded 100 parts by weight, there is a problem wherein the efficiency of the catalyst is decreasing since concentration of the precursors is relatively low.

The material in said mixing step may further contain sulfur component therein. Said sulfur component plays a role in supplementing sulfur deficient when $MoS_2$ is produced during the reacting step.

Said sulfur component may include at list one selected from the group comprising DMDS, S, DBT, butanethiol, and $NH_4CNS$.

Said sulfur component may be added 0.01 to 100 parts by weight for 1 part by weight of the precursor. When said sulfur component is added less than 0.01 parts by weight for 1 part by weight of the precursor, there is a problem wherein a sufficient effect according to the addition of the sulfur component is difficult to obtain; if it is exceeded 100 parts by weight, there is a problem wherein the preparation unit cost of the catalyst is increasing due to the waste of material.

Said reacting step is for preparing a catalyst by disposing said material mixed in said mixing step inside the reactor, and letting it react under the high pressure $H_2$ gas environment of 10 bar to 200 bar.

Said reacting step may be the one, wherein the pressure inside the reactor is maintained 10bar to 200bar under the $H_2$ gas environment, then the temperature is raised to 300-400°C, and subjected to reaction for 2-4 hours.

In said reaction step, the precursors in the form of $MoS_4$ are transformed into $MoS_3$ at the temperature about 200°C during the heating process, and transformed into $MoS_2$ as the temperature rises further. At this moment, supplying $H_2$ plays a role in increasing the conversion efficiency into $MoS_2$ by transforming S into a form of $H_2S$.

At this moment, various methods may be used for creating a $H_2$ gas environment inside the reactor.

According to an exemplary embodiment of the present invention, said reacting step may be the one, wherein $H_2$ gas is allowed to flow continuously passing through the inside of the reactor using a mass flow meter, and the internal pressure of the reactor is maintained 10bar to 200bar using a back pressure regulator, then the temperature is raised to 300-400°C, and subjected to reaction for 2-4 hours.

At this moment, the flow rate of said $H_2$ gas is preferably 1cc/min to 300cc/min per 5g of precursors.

When said flow rate of said $H_2$ gas is less than 1cc/min per 5g of precursors, there is a problem wherein S may not be removed in a form of $H_2S$; if it is exceeded 300cc/min per 5g of precursors, there is a problem wherein the production of $MoS_2$ may not be smooth by removing too much S.

According to another exemplary embodiment of the present invention, said reacting step may be the one, wherein $H_2$ gas is allowed to flow continuously passing through the inside of the reactor using a check valve, and the internal pressure of the reactor is maintained 10bar to 200bar using a back pressure regulator, then the temperature is raised to 300-400°C, and subjected to reaction for 2-4 hours.

According to yet another exemplary embodiment of the present invention, said reacting step may be the one, wherein $H_2$ gas is being injected into the inside of the reactor, and the pressure is maintained 10bar to 200bar, then the temperature is raised to 300-400°C, and subjected to reaction for 2-4 hours.

In said reacting step, it is preferred that $H_2$ gas is allowed to flow continuously passing through the inside of the reactor using a mass flow meter or a check valve. In such a way, when the reaction is performed while $H_2$ gas is flowing continuously passing through the inside of the reactor using a mass flow meter or a check valve, sufficient $H_2$ can be supplied compared to the reaction being performed with $H_2$ gas which is initially injected into the reactor; thus, the produced $H_2S$ is continuously exhausted to the outside of the reactor; therefore the production of $H_2S$ inside the reactor is promoted according to the chemical equilibrium principle, thereby increasing the production efficiency of $MoS_2$.

After the reaction is completed, the temperature is decreased to room temperature while ventilating with $N_2$. For example, said ventilation may be the one wherein the residual gas inside the reactor is removed by ventilation wherein $N_2$ is being passed through the reactor with the condition(flow rate) of 100cc/min to 200cc/min, for 1-2 hours.

When the reaction is completed, the catalyst, prepared after the completion of the reaction, may undergo a pulverizing step under the high pressure according to necessity. After going through such a step, since the specific surface area is enlarged, the methanation efficiency may be increased. At this moment, the pulverizing pressure is preferable to be 5bar to 40,000bar; the grain size of the pulverizing is preferable to be less than 10 mesh (2.00 mm).

Also the pulverized catalyst may additionally filled inside the reactor, then the residual gas inside the reactor may be removed through ventilation wherein $N_2$ is being passed through the reactor with the condition(flow rate) of 100cc/min to 200cc/min, for 1-2 hours.

When the reaction is completed, it goes through an activating step; said activating step is for activating the catalyst which is prepared in said reacting step.

Said activating step may be the one wherein the temperature of the reactor is heated up to 400°C to 500°C with a speed of more than 30°C/min.

Preferably said activating step may be the one wherein it is maintained at least 5 hours at the above described temperature while 0.3% of $H_2S/H_2$ (residual amount) gas is being flowed through. When the activation is completed, the gas is replaced by nitrogen and exhausted through ventilation until it is reached to room temperature.

The catalyst, which the activation thereof is completed, may be formed by compression under the high pressure, and the forming can be done into proper shapes suitable for applications thereof.

A direct methanation catalyst which is prepared through the above described method shows increase in dispersivity and specific surface area, high reaction activation, poison resistance, and reaction durability, thus the direct synthetic gas methanation efficiency may be improved. In addition, since there is no need for a separate installation of an apparatus for removing acid gases and a water gas reactor at the front end of the methanation process, more than 30% in process installation and operation cost can be reduced.

Hereinafter the contents of the present invention will be described in detail using the exemplary embodiments and experiments. However, these are for a more detailed description of the present invention, the scope of the present invention is not limited by these.

<Exemplary embodiment 1>

After pulverizing ammonium tetrathiomolybdate (ATM), 99.99%, $(NH_4)_2MoS_4$, which is a precursor, into a powder, a pressure of 10,000bar was applied for 10 min, then it was repulverized.

After mixing 1.6g of the pulverized specimen with 5g of water, which is a solvent, and 19g of decalin, $H_2$ gas was injected and while the initial pressure was maintained at 15bar the temperature was increased to 350°C; then reaction was performed while maintaining this temperature for 3 hours. After the reaction was completed, the temperature was reduced to room temperature while ventilating using $N_2$, and the pressure was slowly reduced to normal pressure.

Later, it was disposed inside the compressor and compressed with 10,000atm, then pulverized into the size of 6-8 mesh (2.36 to 3.35 mm). After the pulverized catalyst was filled inside the atmospheric pressure reactor, the existing gas inside the reactor was removed by ventilation wherein $N_2$ was being passed through the reactor with the condition(flow rate) of 100cc/min, for 1 hour; then, the direct methanation catalyst was prepared by heating the temperature of the reactor up to 450°C at a speed of 30°C/min.

For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 2>

After pulverizing ammonium tetrathiomolybdate (ATM), 99.99%, $(NH_4)_2MoS_4$, which is a precursor, into a powder, a pressure of 10,000bar was applied for 10 min, then it was repulverized.

After mixing 1.6g of the pulverized specimen with 5g of water, which is a solvent, and 5g of DMDS, which is a sulfur component, $H_2$ gas was injected and while the initial pressure was maintained at 15bar the temperature was increased to 350°C; then reaction was performed while maintaining this temperature for 3 hours. After the reaction was completed, the temperature was reduced to room temperature while ventilating using $N_2$, and the pressure was slowly reduced to normal pressure, then the catalyst was collected.

Later, it was disposed inside the compressor and compressed with 10,000atm, then pulverized into the size of 6-8 mesh (2.36 to 3.35 mm). After the pulverized catalyst was filled inside the atmospheric pressure reactor, the existing gas inside the reactor was removed by ventilation wherein $N_2$ was being passed through the reactor with the condition(flow rate) of 100cc/min, for 1 hour; then, the direct methanation catalyst was prepared by heating the temperature of the reactor up to 450°C at a speed of 30°C/min.

For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 3>

After pulverizing ammonium tetrathiomolybdate (ATM), 99.99%, $(NH_4)_2MoS_4$, which is a precursor, into a powder, a pressure of 10,000bar was applied for 10 min, then it was repulverized.

After mixing 1.6g of the pulverized specimen with 0.24g $ZrNO_3$, which is a co-catalyst precursor, and 150cc of tetralin, which is a solvent, $H_2$ gas was injected and while the initial pressure was maintained at 15bar the temperature was increased to 350°C; then reaction was performed while maintaining this temperature for 3 hours. After the reaction was completed, the temperature was reduced to room temperature while ventilating using $N_2$, and the pressure was slowly reduced to normal pressure, then the catalyst was collected.

Later, it was disposed inside the compressor and compressed with 10,000atm, then pulverized into the size of 6-8 mesh (2.36 to 3.35 mm). After the pulverized catalyst was filled inside the atmospheric pressure reactor, the existing gas inside the reactor was removed by ventilation wherein $N_2$ was being passed through the reactor with the condition(flow rate) of 100cc/min, for 1 hour; then, the direct methanation catalyst was prepared by heating the temperature of the reactor up to 450°C at a speed of 30°C/min, and being left as it is for 10 min.

For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached, and pulverized into a powder and compressed by 10,000atm, then, by forming thereof, a direct methanation catalyst was prepared.

<Exemplary embodiment 4>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas was being flowed continuously at the rate of 30cc/min using a mass flow meter; while the pressure was maintained at 15bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 5>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas was being flowed continuously at the rate of 50cc/min using a mass flow meter; while the pressure was maintained at 15bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 6>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas was being flowed continuously at the rate of 100cc/min using a mass flow meter; while the pressure was maintained at 15bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 7>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas was being flowed continuously at the rate of 200cc/min using a mass flow meter; while the pressure was maintained at 15bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 8>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas was being flowed continuously at the rate of 300cc/min using a mass flow meter; while the pressure was maintained at 15bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 9>

Ammonium tetrathiomolybdate (ATM), 99.99%, $(NH_4)_2MoS_4$, which is a precursor, was pulverized into a powder.
5g of the pulverized specimen and 250g of water, which is a solvent, were put into a reaction container having an internal volume of 1 liter; $H_2$ gas was being flowed continuously at the rate of 390cc/min using a mass flow meter; while the pressure was maintained at 15bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.
Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 10>

Ammonium tetrathiomolybdate (ATM), 99.99%, $(NH_4)_2MoS_4$, which is a precursor, was pulverized into a powder.
5g of the pulverized specimen and 250g of water, which is a solvent, were put into a reaction container having an internal volume of 1 liter; $H_2$ gas was being flowed continuously at the rate of 50cc/min using a mass flow meter; while the pressure was maintained at 20bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.
Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 11>

Ammonium tetrathiomolybdate (ATM), 99.99%, $(NH_4)_2MoS_4$, which is a precursor, was pulverized into a powder.
5g of the pulverized specimen and 250g of water, which is a solvent, were put into a reaction container having an internal volume of 1 liter; $H_2$ gas was being flowed continuously at the rate of 30cc/min using a mass flow meter; while the pressure was maintained at 30bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.
Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 12>

Ammonium tetrathiomolybdate (ATM), 99.99%, $(NH_4)_2MoS_4$, which is a precursor, was pulverized into a powder.
5g of the pulverized specimen and 250g of water, which is a solvent, were put into a reaction container having an internal volume of 1 liter; $H_2$ gas was being flowed continuously at the rate of 70cc/min using a mass flow meter; while the pressure was maintained at 30bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.
Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 13>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas was being flowed continuously at the rate of 30cc/min using a mass flow meter; while the pressure was maintained at 40bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 14>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas was being flowed continuously at the rate of 70cc/min using a mass flow meter; while the pressure was maintained at 40bar using a back pressure regulator, the temperature was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 15>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas, which was passed through the check valve, was being flowed continuously; while the pressure inside the reactor was maintained at 40bar using a back pressure regulator, the temperature inside the reactor was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Exemplary embodiment 16>

Ammonium tetrathiomolybdate (ATM), 99.99%, $(NH_4)_2MoS_4$, which is a precursor, was pulverized into a powder.

5g of the pulverized specimen and 250g of water, which is a solvent, were put into a reaction container having an internal volume of 1 liter; $H_2$ gas, which was passed through the check valve, was being flowed continuously; while the pressure inside the reactor was maintained at 5bar using a back pressure regulator, the temperature inside the reactor was increased to 350°C, which was being maintained thereafter; and it was subjected to reaction for 2 hours. After the reaction was completed, the temperature was reduced to room temperature while ventilating using $N_2$, and the pressure was slowly reduced to normal pressure, then the catalyst was collected.

Later, the collected catalyst was pulverized into a size of 6-8 mesh (2.36 to 3.35 mm). For activation, the catalyst was maintained 5 hours at 450°C while 0.3% of $H_2S/H_2$ (residual amount) gas was being flowed through. Later, after the gas was replaced by nitrogen, it was exhausted through ventilation until room temperature was reached; then, a direct methanation catalyst was prepared.

<Experiment 1>

1g of catalyst prepared in the above described Exemplary embodiment 1 was filled into an Inconel 600 reaction container having an internal diameter of 8mm; after the reduction for 3 hours at 400°C using $H_2$ at a flow rate of 150cc/min the reaction temperature was maintained at 400°C; and a GHSV of 4,800/hr was maintained by injecting a reactive gas comprising 40.5 vol% CO, 45 vol% $H_2$, 0.1 vol% $H_2S$, and 14.4 vol% Ar. The gas concentration at the exit of the reactor was 17.1 vol% $CH_4$, 19.8 vol% $H_2$, 20.5 vol% CO, and 16.1 vol% $CO_2$, and the CO conversion rate was 62.5%.

<Experiment 2>

1g of catalyst prepared in the Exemplary embodiment 2 was filled into an Inconel 600 reaction container having an internal diameter of 8mm; after the reduction for 3 hours at 400°C using $H_2$ at a flow rate of 150cc/min the reaction temperature was maintained at 400°C; and a GHSV of 4,800/hr was maintained by injecting a reactive gas comprising 33.45 vol% CO, 50.41 vol% $H_2$, 14.13 vol% $CH_4$, and 2.01 vol% Ar. The gas concentration at the exit of the reactor was 32.4 vol% $CH_4$, 31.5 vol% $H_2$, 18.0 vol% CO, and 12.2 vol% $CO_2$, and the CO conversion rate was 60.1%.

<Experiment 3>

1g of catalyst prepared in the Exemplary embodiment 3 was filled into an Inconel 600 reaction container having an internal diameter of 8mm; after the reduction for 3 hours at 400°C using $H_2$ at a flow rate of 150cc/min the reaction temperature was maintained at 400°C; and a GHSV of 4,800/hr was maintained by injecting a reactive gas comprising 45.0 vol% CO, 44.75 vol% $H_2$, 0.3 vol% $H_2S$, and 9.95 vol% Ar. The gas concentration at the exit of the reactor was 25.5 vol% $CH_4$, 17.1 vol% $H_2$, 22.9 vol% CO, and 25.6 vol% $CO_2$, and the CO conversion rate was 70.1%.

<Experiment 4>

1g of catalyst prepared in the above described Exemplary embodiment 4 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 37.0 vol% $CH_4$, 7.5 vol% $H_2$, 18.7 vol% CO, and 34.3 vol% $CO_2$, and the CO conversion rate was 80.3%.

<Experiment 5>

1g of catalyst prepared in the Exemplary embodiment 5 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 36.6 vol% $CH_4$, 7.4 vol% $H_2$, 15.4 vol% CO, and 36.6 vol% $CO_2$, and the CO conversion rate was 83.8%.

<Experiment 6>

1g of catalyst prepared in the Exemplary embodiment 6 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 28.9 vol% $CH_4$, 13.4 vol% $H_2$, 27.1 vol% CO, and 27.9 vol% $CO_2$, and the CO conversion rate was 69.5%.

<Experiment 7>

1g of catalyst prepared in the Exemplary embodiment 7 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 30.7 vol% $CH_4$, 11.7 vol% $H_2$, 50.0 vol% CO, and 10.0 vol% $CO_2$, and the CO conversion rate was 73.6%.

<Experiment 8>

1g of catalyst prepared in the Exemplary embodiment 8 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 14.9 vol% $CH_4$, 27.4 vol% $H_2$, 40.1 vol% CO, and 15.9 vol% $CO_2$, and the CO conversion rate was 44.4%.

<Experiment 9>

1g of catalyst prepared in the Exemplary embodiment 9 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The CO conversion rate at the exit of the reactor was 11.2%.

<Experiment 10>

1g of catalyst prepared in the Exemplary embodiment 10 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 39.7 vol% $CH_4$, 5.4 vol% $H_2$, 13.5 vol% CO, and 38.4 vol% $CO_2$, and the CO conversion rate was 86.2%.

<Experiment 11>

1g of catalyst prepared in the Exemplary embodiment 11 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 39.8 vol% $CH_4$, 5.0 vol% $H_2$, 11.7 vol% CO, and 39.8 vol% $CO_2$, and the CO conversion rate was 88.1%.

<Experiment 12>

1g of catalyst prepared in the Exemplary embodiment 12 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 36.3 vol% $CH_4$, 7.3 vol% $H_2$, 16.7 vol% CO, and 36.0 vol% $CO_2$, and the CO conversion rate was 82.8%.

<Experiment 13>

1g of catalyst prepared in the Exemplary embodiment 13 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 31.7 vol% $CH_4$, 10.6 vol% $H_2$, 21.7 vol% CO, and 32.4 vol% $CO_2$, and the CO conversion rate was 76.7%.

<Experiment 14>

1g of catalyst prepared in the Exemplary embodiment 14 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The gas concentration at the exit of the reactor was 22.2 vol% $CH_4$, 19.0 vol% $H_2$, 33.0 vol% CO, and 22.9 vol% $CO_2$, and the CO conversion rate was 60.8%.

<Experiment 15>

1g of catalyst prepared in the Exemplary embodiment 15 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The CO conversion rate at the exit of the reactor was 95.2%.

<Experiment 16>

1g of catalyst prepared in the Exemplary embodiment 16 was filled into an Inconel 600 reaction container having an internal diameter of 8mm; the reaction temperature was maintained at 400°C; and a GHSV of 4,800/hr was maintained by injecting a reactive gas, comprising 50.0 vol% CO, 10.0 vol% $H_2$, at a flow rate of 80cc/min using a mass flow meter. The CO conversion rate at the exit of the reactor was 57.7%.

As confirmed by the forgoing Experiments 1 to 16, it can be confirmed that a direct methanation catalyst prepared by the method of the present invention has a very high CO conversion rate.

The Exemplary embodiments 1 to 3 are corresponding to the cases wherein the reactions were performed while $H_2$ was being supplied to the inside of the reactor; it can be found that the CO conversion rate is especially high in the Exemplary embodiment 3

The Exemplary embodiments 4 to 14 are corresponding to the cases wherein $H_2$ was being continuously flowed using a mass flow meter; it can be found that the CO conversion rate is rapidly decreasing in the Exemplary embodiments 8 and 9 wherein the flow rate of $H_2$ exceeds 300cc/min. Thus, it can be found that the flow rate for a case wherein $H_2$ is being continuously flowed using a mass flow meter is 30cc/min to 200cc/min.

The Exemplary embodiments 15 and 16 are corresponding to the cases wherein $H_2$ was being continuously flowed using a check valve; it can be found that the CO conversion rate in the Exemplary embodiments 15, which uses high pressure, is 95.2%, which is very high.

Thus, it can be found that when preparing a $MoS_2$ series direct methanation catalyst, a superior catalyst can be prepared when $H_2$ gas is being flowed using a check valve or a mass flow meter under the elevated pressure. In addition, it can be found that when preparing a catalyst, a catalyst exhibiting a more superior CO conversion rate can possibly be prepared if a co-catalyst is added thereto.

**Claims**

1.  A method for preparing a direct methanation catalyst for a synthetic gas, as a method for preparing molybdenum disulfide ($MoS_2$) series direct methanation catalyst, being **characterized in that** and including the steps of:

    pulverizing step for pulverizing precursors containing sulfur and molybdenum to a size less than 10 mesh (2.00 mm);
    mixing step for mixing a material containing solvent into the precursors pulverized in said pulverizing step;
    reacting step for preparing a catalyst by disposing said material mixed in said mixing step inside a reactor, and letting it react under the high pressure $H_2$ gas environment of 10 bar to 200 bar; and
    activating step for activating the prepared catalyst for activation thereof,
    wherein the solvent is added 0.01 to 100 parts by weight for 1 part by weight of the precursor.

2.  A method for preparing a direct methanation catalyst for a synthetic gas according to claim 1, being **characterized in that** said reacting step allows $H_2$ gas to flow continuously passing through the inside of the reactor using a mass flow meter, and after the internal pressure of said reactor is maintained 10 bar to 200 bar using a back pressure regulator, the temperature is raised to 300-400°C, and being subjected to reaction for 2-4 hours.

3.  A method for preparing a direct methanation catalyst for a synthetic gas according to claim 2, being **characterized in that** the flow rate of said $H_2$ gas is 1 cc/min to 300 cc/min per 5 g of precursors.

4.  A method for preparing a direct methanation catalyst for a synthetic gas according to claim 1, being **characterized in that** said reacting step allows $H_2$ gas to flow continuously passing through the inside of the reactor using a check valve, and after the internal pressure of the reactor is maintained 10 bar to 200 bar using a back pressure regulator, the temperature is raised to 300-400°C, and being subjected to reaction for 2-4 hours.

5.  A method for preparing a direct methanation catalyst for a synthetic gas according to claim 1, being **characterized in that** said reacting step injects $H_2$ gas into the inside of the reactor, and after the pressure is maintained 10 bar to 200 bar, the temperature is raised to 300-400°C, and being subjected to reaction for 2-4 hours.

6. A method for preparing a direct methanation catalyst for a synthetic gas according to any one of claims 1 to 5, being **characterized in that** said precursor is ammonium tetrathiomolybdate (ATM).

7. A method for preparing a direct methanation catalyst for a synthetic gas according to any one of claims 1 to 5, being **characterized in that** said solvent includes at least one selected from the group including water, cyclohexane, acetone, heptane, hexadecane, methylnaphthalene, decaline, and tetralin.

8. A method for preparing a direct methanation catalyst for a synthetic gas according to any one of claims 1 to 5, being **characterized in that** material in said mixing step further includes a co-catalyst.

9. A method for preparing a direct methanation catalyst for a synthetic gas according to claim 8, being **characterized in that** said co-catalyst is a metal or a metal compound selected from the group including Zr, Sm, Ce, Yr, Yb, Si, and Al.

10. A method for preparing a direct methanation catalyst for a synthetic gas according to claim 8, being **characterized in that** said co-catalyst is added 0.01 to 20 parts by weight for 1 part by weight of the precursor.

11. A method for preparing a direct methanation catalyst for a synthetic gas according to any one of claims 1 to 5, being **characterized in that** said pulverizing step coarsely grinds said precursor, then a pressure of 5 bar to 40,000 bar is being applied thereon, and then repulverizes it into the size of less than 10 mesh (2.00 mm).

12. A method for preparing a direct methanation catalyst for a synthetic gas according to any one of claims 1 to 5, being **characterized in that** said material in said mixing step further contains sulfur component therein.

13. A method for preparing a direct methanation catalyst for a synthetic gas according to claim 12, being **characterized in that** said sulfur component includes at list one selected from the group including DMDS, S, DBT, butanethiol, and $NH_4CNS$.

14. A method for preparing a direct methanation catalyst for a synthetic gas according to claim 12, being **characterized in that** said sulfur component is added 0.01 to 100 parts by weight for 1 part by weight of said precursor.

**Patentansprüche**

1. Verfahren zur Herstellung eines Direktmethanisierungskatalysators für ein synthetisches Gas als ein Verfahren zur Herstellung eines Direktmethanisierungskatalysators der Molybdändisulfid (MoS2)-Serie, **dadurch gekennzeichnet, dass** und die folgenden Schritte umfassend:

   Pulverisierungsschritt zum Pulverisieren von schwefel- und molybdänhaltigen Vorläufern auf eine Größe von weniger als 10 mesh (2,00 mm);
   Mischschritt zum Mischen eines lösungsmittelhaltigen Materials in die in dem Pulverisierungsschritt pulverisierten Vorläufer;
   Reaktionsschritt zur Herstellung eines Katalysators durch Anordnen des in dem Mischschritt gemischten Materials in einem Reaktor und Reagierenlassen unter dem Hochdruck $H_2$-Gasumfeld von 10 bar bis 200 bar; und Aktivierungsschritt zum Aktivieren des hergestellten Katalysators zum Aktivieren desselben,
   wobei dem Lösungsmittel 0,01 bis 100 Gewichtsteile für 1 Gewichtsteil des Vorläufers zugegeben werden.

2. Verfahren zur Herstellung eines Direktmethanisierungskatalysators für ein synthetisches Gas gemäß Anspruch 1, **dadurch gekennzeichnet, dass** der Reaktionsschritt es dem $H_2$-Gas ermöglicht, kontinuierlich durch das Innere des Reaktors unter Verwendung eines Massenflussmessers zu strömen, und nachdem der Innendruck des Reaktors unter Verwendung eines Gegendruckreglers auf 10 bar bis 200 bar gehalten wird, die Temperatur auf 300-400°C erhöht wird und einer Reaktion für 2-4 Stunden ausgesetzt wird.

3. Verfahren zur Herstellung eines Direktmethanisierungskatalysators für ein synthetisches Gas gemäß Anspruch 2, **dadurch gekennzeichnet, dass** die Durchflussrate des $H_2$-Gases 1 cc/min bis 300 cc/min pro 5 g Vorläufer beträgt.

4. Verfahren zur Herstellung eines Direktmethanisierungskatalysators für ein synthetisches Gas gemäß Anspruch 1, **dadurch gekennzeichnet, dass** der Reaktionsschritt es dem $H_2$-Gas ermöglicht, kontinuierlich durch das Innere des Reaktors unter Verwendung eines Rückschlagventils zu strömen, und nachdem der Innendruck des Reaktors unter Verwendung eines Gegendruckreglers auf 10 bar bis 200 bar gehalten wird, die Temperatur auf 300-400°C erhöht wird, und einer Reaktion für 2-4 Stunden ausgesetzt wird.

5. Verfahren zur Herstellung eines Direktmethanisierungskatalysators für ein synthetisches Gas gemäß Anspruch 1, **dadurch gekennzeichnet, dass** der Reaktionsschritt $H_2$-Gas in das Innere des Reaktors einspritzt und, nach der Aufrechterhaltung des Drucks auf 10 bar bis 200 bar, die Temperatur auf 300-400°C erhöht wird, und einer Reaktion für 2-4 Stunden ausgesetzt wird.

6. Verfahren zur Herstellung eines Direktmethanisierungskatalysators für ein synthetisches Gas gemäß einem der Ansprüche 1 bis 5, **dadurch gekennzeichnet, dass** der Vorläufer Ammoniumtetrathiomolybdat (ATM) ist.

7. Verfahren zur Herstellung eines Direktmethanisierungskatalysators für ein synthetisches Gas gemäß einem der Ansprüche 1 bis 5, **dadurch gekennzeichnet, dass** das Lösungsmittel mindestens eines aus der Gruppe Wasser, Cyclohexan, Aceton, Heptan, Hexadecan, Methylnaphthalin, Decalin und Tetralin umfasst.

8. Verfahren zur Herstellung eines Direktmethanisierungskatalysators für ein synthetisches Gas gemäß einem der Ansprüche 1 bis 5, **dadurch gekennzeichnet, dass** das Material in dem Mischschritt ferner einen Co-Katalysator umfasst.

9. Verfahren zur Herstellung eines Direktmethanisierungskatalysators für ein synthetisches Gas gemäß Anspruch 8, **dadurch gekennzeichnet, dass** der Co-Katalysator ein Metall oder eine Metallverbindung ist, der aus der Gruppe, die Zr, Sm, Ce, Yr, Yb, Si und Al umfasst, ausgewählt ist.

10. Verfahren zur Herstellung eines Direktmethanisierungskatalysators für ein synthetisches Gas gemäß Anspruch 8, **dadurch gekennzeichnet, dass** dem Co-Katalysator 0,01 bis 20 Gewichtsteile für 1 Gewichtsteil des Vorläufers zugegeben werden.

11. Verfahren zur Herstellung eines Direktmethanisierungskatalysators für ein synthetisches Gas gemäß einem der Ansprüche 1 bis 5, **dadurch gekennzeichnet, dass** der Pulverisierungsschritt den Vorläufer grob mahlt, dann ein Druck von 5 bar bis 40.000 bar darauf aufgebracht wird und ihn dann auf die Größe von weniger als 10 mesh (2,00 mm) re-pulverisiert.

12. Verfahren zur Herstellung eines Direktmethanisierungskatalysators für ein synthetisches Gas gemäß einem der Ansprüche 1 bis 5, **dadurch gekennzeichnet, dass** das Material in dem Mischschritt ferner eine Schwefelkomponente darin enthält.

13. Verfahren zur Herstellung eines Direktmethanierungskatalysators für ein synthetisches Gas nach Anspruch 12, **dadurch gekennzeichnet, dass** die Schwefelkomponente mindestens eine aus der Gruppe bestehend aus DMDS, S, DBT, Butanethiol und $NH_4CNS$ umfasst,

14. Verfahren zur Herstellung eines Direktmethanisierungskatalysators für ein synthetisches Gas gemäß Anspruch 12, **dadurch gekennzeichnet, dass** der Schwefelkomponente 0,01 bis 100 Gewichtsteile für 1 Gewichtsteil des Vorläufers zugegeben werden.

**Revendications**

1. Procédé pour préparer un catalyseur de méthanisation directe pour un gaz synthétique, en tant que procédé pour préparer un catalyseur de méthanisation directe en série de disuffure de molybdène ($MoS_2$), qui est **caractérisé en ce que** et comportant les étapes consistant en :

   une étape de pulvérisation pour pulvériser des précurseurs contenant du soufre et du molybdène pour obtenir une taille inférieure à 10 mesh (2,00 mm) ;
   une étape de mélange pour mélanger un matériau contenant un solvant dans les précurseurs pulvérisés lors de ladite étape de pulvérisation ;
   une étape de réaction pour préparer un catalyseur en disposant ledit matériau mélangé lors de ladite étape de mélange à l'intérieur d'un réacteur, et le laisser réagir dans l'environnement de gaz $H_2$ sous forte pression de 10 bar à 200 bar; et

une étape d'activation pour activer le catalyseur préparé pour une activation de celui-ci,
dans lequel le solvant est ajouté à raison de 0,01 à 100 parties en poids pour 1 partie en poids du précurseur.

2. Procédé pour préparer un catalyseur de méthanisation directe pour un gaz synthétique selon la revendication 1, qui est **caractérisé en ce que** ladite étape de réaction permet à un gaz $H_2$ de s'écouler de manière continue en passant à travers l'intérieur du réacteur à l'aide d'un débitmètre massique, et après que la pression interne dudit réacteur a été maintenue de 10 bar à 200 bar à l'aide d'un régulateur de contre-pression, la température est accrue à 300 à 400 °C, et qui est soumis à une réaction pendant 2 à 4 heures,

3. Procédé pour préparer un catalyseur de méthanisation directe pour un gaz synthétique selon la revendication 2, qui est **caractérisé en ce que** le débit dudit gaz $H_2$ est de 1 cm³/min à 300 cm³/min pour 5 g de précurseurs.

4. Procédé pour préparer un catalyseur de méthanisation directe pour un gaz synthétique selon la revendication 1, qui est **caractérisé en ce que** ladite étape de réaction permet à un gaz $H_2$ de s'écouler de manière continue en passant à travers l'intérieur du réacteur à l'aide d'un clapet de non-retour, et après que la pression interne du réacteur a été maintenue de 10 bar à 200 bar à l'aide d'un régulateur de contre-pression, la température est accrue à 300 à 400 °C, et qui est soumis à une réaction pendant 2 à 4 heures.

5. Procédé pour préparer un catalyseur de méthanisation directe pour un gaz synthétique selon la revendication 1, qui est **caractérisé en ce que** ladite étape de réaction injecte un gaz $H_2$ à l'intérieur du réacteur, et après que la pression a été maintenue de 10 bar à 200 bar, la température est accrue à 300 à 400 °C, et qui est soumis à une réaction pendant 2 à 4 heures.

6. Procédé pour préparer un catalyseur de méthanisation directe pour un gaz synthétique selon l'une quelconque des revendications 1 à 5, qui est **caractérisé en ce que** ledit précurseur est le tétrathiomolybdate d'ammonium (ATM).

7. Procédé pour préparer un catalyseur de méthanisation directe pour un gaz synthétique selon l'une quelconque des revendications 1 à 5, qui est **caractérisé en ce que** ledit solvant comporte au moins l'un sélectionné à partir du groupe comportant l'eau, le cyclohexane, l'acétone, l'heptane, l'hexadécane, le méthylnaphtalène, la décaline et la tétraline.

8. Procédé pour préparer un catalyseur de méthanisation directe selon l'une quelconque des revendications 1 à 5, qui est **caractérisé en ce que** le matériau lors de ladite étape de mélange comporte en outre un cocatalyseur.

9. Procédé pour préparer un catalyseur de méthanisation directe pour un gaz synthétique selon la revendication 8, qui est **caractérisé en ce que** ledit cocatalyseur est un métal ou un composé métallique sélectionné à partir du groupe comportant les métaux Zr, Sm, Ce, Yr, Yb, Si et Al.

10. Procédé pour préparer un catalyseur de méthanisation directe pour un gaz synthétique selon la revendication 8, qui est **caractérisé en ce que** ledit cocatalyseur est ajouté à raison de 0,01 à 20 parties en poids pour 1 partie en poids du précurseur.

11. Procédé pour préparer un catalyseur de méthanisation directe pour un gaz synthétique selon l'une quelconque des revendications 1 à 5, qui est **caractérisé en ce que** ladite étape de pulvérisation broie grossièrement ledit précurseur, ensuite une pression de 5 bar à 40 000 bar est appliquée sur celui-ci, et ensuite le pulvérise à nouveau pour obtenir la taille inférieure à 10 mesh (2,00 mm).

12. Procédé pour préparer un catalyseur de méthanisation directe pour un gaz synthétique selon l'une quelconque des revendications 1 à 5, qui est **caractérisé en ce que** ledit matériau lors de ladite étape de mélange contient en outre un constituant au soufre dans celui-ci.

13. Procédé pour préparer un catalyseur de méthanisation directe pour un gaz synthétique selon la revendication 12, qui est **caractérisé en ce que** ledit constituant au soufre comporte au moins l'un sélectionné à partir du groupe comportant les constituants DMDS, S, DBT, butanethiol et $NH_4CNS$.

14. Procédé pour préparer un catalyseur de méthanisation directe pour un gaz synthétique selon la revendication 12, qui est **caractérisé en ce que** ledit constituant au soufre est ajouté à raison de 0,01 à 100 parties en poids pour 1 partie en poids dudit précurseur.

**REFERENCES CITED IN THE DESCRIPTION**

**Patent documents cited in the description**

- US 4243554 A
- WO 2012040111 A